# EUROPEAN PATENT APPLICATION

(11) **EP 4 249 033 A1**
(43) Date of publication of application: **27.09.2023**
(21) Application number: 21893996.5
(22) Date of filing: 19.11.2021
(51) Int. Cl.: A61M 25/10, A61L 29/08, A61L 29/14, A61L 29/16

(54) **DRUG-COATED BALLOON AND PREPARATION METHOD THEREFOR**

(30) Priority: 19.11.2020 CN 202011303535; 25.10.2021 CN 202111242947
(71) Applicant: Shanghai Shenqi Medical Technology Co., Ltd., Shanghai 201203 (CN)
(72) Inventor: WANG, Sen, Shanghai 201203 (CN); YU, Shaoxing, Shanghai 201203 (CN); WANG, Zhanjun, Shanghai 201203 (CN); DAI, Zhihao, Shanghai 201203 (CN); WANG, Dingxi, Shanghai 201203 (CN)
(74) Representative: Cabinet Laurent & Charras
(86) International application number: PCT/CN2021/131619
(87) International publication number: WO 2022/105849

(57) **Abstract**

The present application relates to a drug-coated balloon and a preparation method therefor. The drug-coated balloon includes a balloon body and a drug coating disposed on an outer surface of the balloon body. The drug coating includes a bottom coating, an intermediate coating, and a top coating stacked in sequence. The bottom coating is used to improve the firmness and the adhesion of the drug coating to the balloon body, thereby reducing the drug loss in the delivery process and increasing the drug utilization rate. The intermediate coating is used to carry the drug and assist in drug absorption. The top coating is used to promote the adherence of the drug coating to the tunica intima of the blood vessel, which is more conducive to the drug absorption by the tissue.

## Description

### CROSS-REFERENCE TO RELATED APPLICATIONS

This application claims priorities of China Patent Application No. 202011303535.8, filed on 19 November 2020 with the Chinese patent office, entitled "DRUG-COATED BALLOON AND PREPARATION METHOD THEREFOR" and of China Patent Application No. 202111242947.X, filed on 25 October 2021 with the Chinese patent office, entitled "DRUG-COATED BALLOON AND PREPARATION METHOD THEREFOR", the contents of which are hereby incorporated by reference in their entireties.

### TECHNICAL FIELD

The present application relates to the technical field of medical devices, and in particular to drug-coated balloons and preparation methods therefor.

### BACKGROUND

A drug-coated balloon is a new therapeutic balloon drug-release technology developed on the basis of interventional technology such as balloon dilation or balloon angioplasty. An anti-proliferative drug such as paclitaxel is coated onto the surface of the balloon, so that when the balloon reaches the lesion site and is expanded and dilated to be in contact with the tunica intima of the blood vessel, the drug is rapidly released and transferred locally to the blood vessel wall. The drug in the local area plays a role in inhibiting intima hyperplasia of the blood vessel, thereby preventing vascular restenosis after the vascular intervention. By using the drug-coated balloon, on the one hand, the drug can effectively inhibit hyperplasia of smooth muscle cells, thereby reducing the incidence of restenosis; on the other hand, there is no need to implant a stent, thereby reducing inflammatory reaction of the tunica intima, lowering the risk of in-stent thrombosis, shortening the duration of dual antiplatelet therapy, and reducing the risk of bleeding. In addition, in clinical studies of coronary artery diseases, the drug-coated balloons have shown better efficacy and safety in treating in-stent restenosis, small vessel disease, and bifurcation lesions. The drug-coated balloons are also suitable for patients at high bleeding risk, taking oral anticoagulants, or recently received surgery.

Drug coating as an important technical indicator is crucial to the drug-coated balloon. The firmness of the drug coating in the blood vessel, the drug releasability in the artery, and the drug absorbability by the blood vessel are all determined by the drug coating. From the currently available patent documents and literatures, it can be seen that the firmness of the drug coating is not high, and there is a significant drug loss during the delivery and expansion of the drug-coated balloon, resulting in a low drug utilization rate. In order to reduce the drug loss during the delivery, some coating technologies offer a protective film outside the coating. However, the protective film is too impervious such that the drug is unable to be in direct contact with the blood vessel wall. The absorption of the drug by tissues is also adversely affected by the over small contact area.

While the drug coating can release drug rapidly, a significant amount of drug will be washed away by blood in the delivery process, leaving very little drug at the local area. The local drug absorption can be improved by increasing the loading amount of the drug in the coating on the balloon, but a large amount of excess drug will flow to the distal end of the blood vessel, affecting the patient's health. The drug is difficult to be released considering the short expansion time of an ordinary drug-coated balloon dilation catheter. Thus, the ordinary drug-coated balloon dilation catheter currently has the following problems and challenges: (1) While the drug coating releases the drug rapidly, a significant amount of the drug can be washed away by the blood in the delivery process; (2) the drug adheres to the blood vessel wall for a short period of time and thus is difficult to be absorbed in large quantity, thereby failing to achieve a desired therapeutic effect; (3) the drug coating is prone to shedding particles, which can lead to thrombosis.

### SUMMARY

According to embodiments of the present application, a drug-coated balloon and a preparation method therefor are provided. The drug-coated balloon has high coating firmness, desired drug release ability, and good drug absorption by the blood vessel.

The following technical solutions are adopted to achieve the object of the present application.

A drug-coated balloon includes a balloon body and a drug coating disposed on an outer surface of the balloon body. The drug coating includes a bottom coating, an intermediate coating, and a top coating stacked in sequence.

In an embodiment, the bottom coating includes a polymer material. The intermediate coating includes a drug and a lipophilic material. The top coating includes another polymer material.

In an embodiment, the bottom coating includes any one or a combination of at least two of poly(lactic-co-glycolic acid), polyethylene glycol, povidone, polyvinyl alcohol, methyl cellulose, hydroxymethyl cellulose, hydroxypropyl cellulose, or polycaprolactone. The top coating includes any one or a combination of at least two of dibutyl hydroxy toluene, butyl hydroxy anisole, chitosan, or polycaprolactone.

In an embodiment, the lipophilic material includes any one or a combination of at least two of cholesterol, a phospholipid, or a fatty acid.

In an embodiment, the lipophilic material includes the cholesterol and the phospholipid. A mass ratio of the cholesterol to the phospholipid is in a range from 0.2 to 10.

In an embodiment, the lipophilic material includes the phospholipid and the fatty acid. A mass ratio of the phospholipid to the fatty acid is in a range from 0.2 to 10.

In an embodiment, the lipophilic material includes the cholesterol and the fatty acid. A mass ratio of the cholesterol to the fatty acid is in a range from 0.04 to 100.

In an embodiment, the drug includes any one of or a combination of at least two of sirolimus, a sirolimus derivative, paclitaxel, or a paclitaxel derivative. A mass ratio of the drug to the lipophilic material is in a range from 0.1 to 10. A mass ratio of the polymer material of the bottom coating to the drug is in a range from 0.05 to 20. A mass ratio of the polymer material of the top coating to the drug is in a range from 0.05 to 20.

In an embodiment, the cholesterol includes DC-cholesterol and/or cholesterol. The phospholipid includes any one or a combination of at least two of 1,2-dioleoyl lecithin, dipalmitoyl phosphatidylcholine, distearoyl phosphatidylcholine, dimyristoyl phosphatidylcholine, 1-palmitoyl-2-oleoyl lecithin, distearoyl phosphatidylethanolamine, dioleoyl phosphatidylethanolamine, or dipalmitoyl phosphatidylethanolamine. The fatty acid includes any one or a combination of at least two of palmitic acid, stearic acid, lauric acid, myristic acid, or arachidic acid.

The present application further provides a method for preparing the above-described drug-coated balloon, including steps of:
(1) preparing a bottom coating solution, an intermediate coating solution, and a top coating solution respectively;
(2) spraying the bottom coating solution, the intermediate coating solution, and the top coating solution in sequence onto the surface of the balloon body to form the drug coating.

In an embodiment, in step (2), the spraying is performed by using an ultrasonic atomization spraying device and adopting a multi-layer stack spraying technology.

As compared to the related art, the present application has the following beneficial effects.

The drug-coated balloon provided by the present application includes the drug coating disposed on the outer surface of the balloon body. The drug coating has a sandwich structure, including the bottom coating, the intermediate coating, and the top coating stacked in sequence. The bottom coating is used to improve the firmness and the adhesion of the drug coating to the balloon body, thereby reducing the drug loss in the delivery process and increasing the drug utilization rate. The bottom coating reduces the amount of drug being washed away by blood in the delivery process while the drug can be released rapidly. By including the bottom coating, the drug coating is less likely to shed particles or cause thrombosis. The intermediate coating is used to carry the drug and assist in drug absorption. The top coating is used to promote the adherence of the drug coating to the tunica intima of the blood vessel, which is more conducive to the drug absorption, thereby achieving the effect that the wall-adherent drug can be absorbed by the blood vessel wall in a short time period.

### DETAILED DESCRIPTION

In order to further illustrate technical solutions adopted in the present application and the effects therefrom, the technical solutions of the present application will be further described below in connection with specific embodiments of the present application. However, the present application is not limited to the scope of the following embodiments.

The present application provides a drug-coated balloon, including a balloon body and a drug coating disposed on an outer surface of the balloon body. The drug coating includes a bottom coating, an intermediate coating, and a top coating, which are stacked in sequence on the outer surface of the balloon body. The bottom coating is used to increase the adhesion of the drug coating to the balloon body and improve the firmness of the drug coating. The intermediate coating is used to carry the drug and assist the drug absorption. The top coating is used to enhance the adherence of the coating to the tunica intima, so as to further facilitate the drug absorption.

In some embodiments, a thickness ratio of the bottom coating to the intermediate coating can be in a range from 0.05 to 10. A thickness ratio of the intermediate coating to the top coating can be in a range from 0.01 to 20. Specifically, the thickness ratio of the bottom coating to the intermediate coating can be 0.05, 0.1, 0.5, 1, 2, 3, 4, 5, 6, 7, 8, 9, 10, etc. The thickness ratio of the intermediate coating to the top coating can be 0.01, 0.1, 0.5, 1, 2, 3, 4, 5, 6, 7, 8, 9, 10, 15, 20, etc. Further, a thickness of the intermediate coating can be in a range from 1 µm to 20 µm, such as 1 µm, 2 µm, 4 µm, 5 µm, 8 µm, 10 µm, 12 µm, 14 µm, 15 µm, 16 µm, 18 µm, 20 µm, etc. The other specific values within the above numeric ranges can also be selected and will not be repeated herein.

In some embodiments, the bottom coating includes a polymer material. Preferably, the bottom coating includes any one or a combination of at least two of poly(lactic-co-glycolic acid), polyethylene glycol, povidone, polyvinyl alcohol, methyl cellulose, hydroxymethyl cellulose, hydroxypropyl cellulose, or polycaprolactone. The polymer material in the bottom coating is preferably a high viscosity polymer material, which is conducive to promoting the adherence of the drug coating to the balloon body, so that the drug will be less likely to be washed away by the blood in the delivery process of the drug-coated balloon and the drug utilization rate will be higher.

The bottom coating can include a combination of at least two polymer materials, such as a combination of poly(lactic-co-glycolic acid) and polyethylene glycol, a combination of polyethylene glycol and povidone, a combination of polyvinyl alcohol and methyl cellulose, a combination of poly(lactic-co-glycolic acid), polyethylene glycol, and povidone, a combination of hydroxymethyl cellulose, hydroxypropyl cellulose, and polycaprolactone, etc. Any of other combinations can also be selected and will not be repeated herein.

In some embodiments, the top coating includes a polymer material. Preferably, the top coating includes any one or a combination of at least two of dibutyl hydroxy toluene, butyl hydroxy anisole, chitosan, or polycaprolactone. The polymer material in the top coating is preferably a lipophilic polymer material, which is conducive to promoting the adherence of the drug coating to the tunica intima, thereby further facilitating the drug absorption.

The top coating can include a combination of at least two polymer materials, such as a combination of dibutyl hydroxy toluene and butyl hydroxy anisole, a combination of butyl hydroxy anisole and chitosan, a combination of dibutyl hydroxy toluene and polycaprolactone, etc. Any of other combinations can also be selected and will not be repeated herein.

In some embodiments, the intermediate coating includes a drug and a lipophilic material. The lipophilic material can include any one or a combination of at least two of cholesterol, a phospholipid, or a fatty acid. The lipophilic material which coexists with the drug in the intermediate coating can help to achieve better wall-adherence and absorption of the drug.

The cholesterol can include DC-cholesterol and/or cholesterol. The phospholipid can include any one or a combination of at least two of 1,2-dioleoyl lecithin, dipalmitoyl phosphatidylcholine, distearoyl phosphatidylcholine, dimyristoyl phosphatidylcholine, 1-palmitoyl-2-oleoyl lecithin, distearoyl phosphatidylethanolamine, dioleoyl phosphatidylethanolamine, or dipalmitoyl phosphatidylethanolamine. The fatty acid can include any one or a combination of at least two of palmitic acid, stearic acid, lauric acid, myristic acid, or arachidic acid.

The lipophilic material can be a combination of at least two materials, such as a combination of cholesterol and the phospholipid, a combination of the phospholipid and the fatty acid, a combination of cholesterol and the fatty acid, and a combination of cholesterol, the phospholipid, and the fatty acid. Any of other combinations can also be selected and will not be repeated herein.

Specifically, the lipophilic material can be a combination of 1,2-dioleoyl lecithin and dipalmitoyl phosphatidylcholine, a combination of distearoyl phosphatidylcholine and dimyristoyl phosphatidylcholine, a combination of 1,2-dioleoyl lecithin, 1-palmitoyl-2-oleoyl lecithin, and distearoyl phosphatidylethanolamine, etc. Any of other combinations can also be selected and will not be repeated herein.

The lipophilic material can also be a combination of palmitic acid and stearic acid, a combination of stearic acid and lauric acid, a combination of myristic acid and arachidic acid, a combination of palmitic acid, stearic acid, and lauric acid, etc. Any of other combinations can also be selected and will not be repeated herein.

In the case that the lipophilic material includes both cholesterol and the phospholipid, a mass ratio of the cholesterol to the phospholipid is in a range from 0.2 to 10, such as 0.2, 0.5, 1, 1.5, 2, 3, 4, 5, 6, 7, 8, 9, 10, etc. The other specific values within the above range can also be selected and will not be repeated herein.

Preferably, a mass ratio of the phospholipid to the fatty acid is in a range from 0.2 to 10, such as 0.2, 0.5, 1, 1.5, 2, 3, 4, 5, 6, 7, 8, 9, 10, etc. The other specific values within the above range can also be selected and will not be repeated herein. The other specific values within the above range can also be selected and will not be repeated herein.

In the case that the lipophilic material includes both cholesterol and the fatty acid, a mass ratio of the cholesterol to the fatty acid is in a range from 0.04 to 100, such as 0.04, 0.05, 0.1, 0.5, 1, 5, 10, 20, 50, 60, 70, 100, etc. The other specific values within the above range can also be selected and will not be repeated herein.

The intermediate coating includes the drug. The drug can include any one or a combination of at least two of sirolimus, a sirolimus derivative, paclitaxel, or a paclitaxel derivative. The combination of at least two drugs can be, for example, a combination of sirolimus and the sirolimus derivative, a combination of paclitaxel and the paclitaxel derivative, a combination of the sirolimus derivative and the paclitaxel derivative, a combination of sirolimus and paclitaxel, etc. Any of other combinations can also be selected and will not be repeated herein. The sirolimus derivative can be, for example, everolimus, zotarolimus, etc. The paclitaxel derivative can be, for example, docetaxel, paclitaxel liposome, albumin-bound paclitaxel, etc.

In the intermediate coating, a mass ratio of the drug to the lipophilic material is preferably in a range from 0.1 to 10, such 0.1, 0.2, 0.5, 1, 2, 5, 7, 9, 10, etc. The other specific values within the above range can also be selected and will not be repeated herein. If the proportion of the lipophilic material is further increased, the amount of the pharmaceutical excipients will be excessive, thereby causing adverse reaction. If the proportion of the lipophilic material is further decreased, the softness of the coating will be changed, and the wall-adherence and drug absorption effect will be decreased. A loading amount of the drug in the intermediate coating can be in a range from 0.1 µg/mm² to 8 µg/mm², such as 0.1 µg/mm², 0.2 µg/mm ², 0.5 µg/mm², 1 µg/mm², 2 µg/mm², 3 µg/mm², 4 µg/mm², 5 µg/mm², 6 µg/mm², 7 µg/mm ², 8 µg/mm², etc. The other specific values within the above range can also be selected and will not be repeated herein. A mass ratio of the polymer material in the bottom coating to the drug in the intermediate coating can be 0.05 to 20, such as 0.05, 0.1, 0.2, 0.5, 1, 5, 10, 15, 20, etc. The other specific values within the above range can also be selected and will not be repeated herein. A mass ratio of the polymer material in the top coating to the drug in the intermediate coating can be in a range from 0.05 to 20, such as 0.05, 0.1, 0.2, 0.5, 1, 5, 10, 15, 20, etc. The other specific values within the above range can also be selected and will not be repeated herein.

The present application further provides a method for preparing the above-described drug-coated balloon, including steps of:
(1) preparing a bottom coating solution, an intermediate coating solution, and a top coating solution respectively;
(2) spraying the bottom coating solution, the intermediate coating solution, and the top coating solution in sequence onto the surface of the balloon body to form the drug coating.

In step (1), raw materials for preparing the bottom coating, the intermediate coating, and the top coating are respectively mixed and dissolved with solvents to produce the bottom coating solution, the intermediate coating solution, and the top coating solution. The solvents includes any one or a combination of at least two of methanol, ethanol, acetone, isopropanol, dimethyl sulfoxide, ethyl acetate, acetonitrile, tetrahydrofuran, methylene chloride, n-heptane, n-hexane, cyclohexane, or water. The combination of at least two solvents can be a combination of ethanol and n-heptane, a combination of ethanol, n-heptane, and dimethyl sulfoxide, etc. Any of other combinations can also be selected and will not be repeated herein.

In step (2), the spraying is performed by an ultrasonic atomization spraying device. The ultrasonic atomization spraying device can adopt multi-layer stack spraying technology or other existing spraying technology to perform the spraying operation. In spraying, the ultrasonic atomization spraying device has an ultrasonic power preferably ranged from 0.2 W to 5 W, such as 0.2 W, 0.5 W, 0.8 W, 1 W, 1.5 W, 2 W, 2.5 W, 3 W, 5 W, etc., a temperature preferably ranged from 20°C to 50°C, such as 20°C, 25°C, 30°C, 35°C, 40°C, 45°C, 50°C, etc., a pressure preferably ranged from 0.01 MPa to 0.3 MPa, such as 0.01 MPa, 0.05 MPa, 0.1 MPa, 0.15 MPa, 0.2 MPa, 0.25 MPa, 0.3 MPa, etc. Other specific point values within the above numerical ranges can also be selected and will not be repeated herein.

Further, a spraying range of the ultrasonic atomization spraying device during the spraying is preferably ranged from 10 mm to 60 mm, such as 10 mm, 20 mm, 30 mm, 40 mm, 50 mm, 60 mm, etc. A flow rate of the drug solution is ranged from 0.01 mL/min to 1 mL/min, such as 0.01 mL/min, 0.02 mL/min, 0.05 mL/min, 0.08 mL/min, 0.1 mL/min, 0.2 mL/min, 0.5 mL/min, 0.8 mL/min, 1.0 mL/min, etc. Other specific point values within the above ranges can be selected and will not be repeated herein.

### Example 1

This example provides a drug-coated balloon and a preparation method therefor. The drug-coated balloon includes a balloon body and a drug coating disposed on an outer surface of the balloon body. The drug coating includes a bottom coating, an intermediate coating, and a top coating, which are stacked in sequence. The thicknesses of the bottom coating, the intermediate coating, and the top coating are respectively 4 µm, 6 µm, and 4 µm.

A raw material for preparing the bottom coating is hydroxymethyl cellulose. A raw material for preparing the top coating is butyl hydroxy anisole. A raw material for preparing the intermediate coating includes sirolimus and a lipophilic material (DC-cholesterol, 1, 2-dioleyl lecithin, and stearic acid).

A mass ratio of DC-cholesterol to 1, 2-dioleyl lecithin to stearic acid is 2:1:1. A mass ratio of sirolimus to the lipophilic material is 1:1. A mass ratio of hydroxymethyl cellulose to sirolimus is 1:1.5. A mass ratio of butyl hydroxy anisole to sirolimus is 1:1.5. A loading amount of sirolimus in the intermediate coating is 3 µg/mm².

The preparation method for the drug-coated balloon is as follows.
(1) Hydroxymethyl cellulose is dissolved in ethanol to form a colorless transparent solution, i.e., a bottom coating solution.
(2) DC-cholesterol, 1,2-dioleyl lecithin, stearic acid, and sirolimus are dissolved in n-heptane and sonicated to form a white suspension liquid, i.e., an intermediate coating solution.
(3) Butyl hydroxy anisole is dissolved in dimethyl sulfoxide to form a transparent solution, i.e., a top coating solution.
(4) The outer surface of the balloon body is sprayed with the bottom coating solution for two times, the intermediate coating solution for six times, and the top coating solution for two times in sequence by using an ultrasonic atomization spraying device, wherein the ultrasonic power is 3.0 W, the flow rate of the drug solution delivered by a syringe pump is 0.08 mL/min, the spraying temperature is 25°C, the spraying pressure is 0.05 MPa, and the spraying range is 30 mm.

### Example 2

This example provides a drug-coated balloon and a preparation method therefor. The drug-coated balloon includes a balloon body and a drug coating disposed on an outer surface of the balloon body. The drug coating includes a bottom coating, an intermediate coating, and a top coating, which are stacked in sequence. The thicknesses of the bottom coating, the intermediate coating, and the top coating are respectively 4 µm, 8 µm, and 4 µm.

A raw material for preparing the bottom coating is hydroxypropyl cellulose. A raw material for preparing the top coating is dibutyl hydroxy toluene. A raw material for preparing the intermediate coating includes sirolimus and a lipophilic material (DC- cholesterol, dipalmitoyl phosphatidylcholine, and lauric acid).

A mass ratio of DC-cholesterol to dipalmitoyl phosphatidylcholine to lauric acid is 1:1:1. A mass ratio of sirolimus to the lipophilic material is 2:1. A mass ratio of hydroxypropyl cellulose to sirolimus is 2:5. A mass ratio of dibutyl hydroxy toluene to sirolimus is 2:5. A loading amount of sirolimus in the intermediate coating is 2 µg/mm².

The preparation method for the drug-coated balloon is as follows.
(1) Hydroxypropyl cellulose is dissolved in ethanol to form a colorless transparent solution, i.e., a bottom coating solution.
(2) DC-cholesterol, dipalmitoyl phosphatidylcholine, lauric acid, and sirolimus are dissolved in n-heptane and sonicated to form a white suspension liquid, i.e., an intermediate coating solution.
(3) Dibutyl hydroxy toluene is dissolved in n-heptane to form a transparent solution, i.e., a top coating solution.
(4) The outer surface of the balloon body is sprayed with the bottom coating solution for two times, the intermediate coating solution for four times, and the top coating solution for four times in sequence by using an ultrasonic atomization spraying device, wherein the ultrasonic power is 5.0 W, the flow rate of the drug solution delivered by a syringe pump is 0.15 mL/min, the spraying temperature is 25°C, the spraying pressure is 0.05 MPa, and the spraying range is 30 mm.

### Example 3

This example provides a drug-coated balloon and a preparation method therefor. The drug-coated balloon includes a balloon body and a drug coating disposed on an outer surface of the balloon body. The drug coating includes a bottom coating, an intermediate coating, and a top coating which are stacked in sequence. The thicknesses of the bottom coating, the intermediate coating, and the top coating are respectively 5 µm, 5 µm, and 5 µm.

A raw material for preparing the bottom coating is a combination of hydroxypropyl cellulose and poly(lactic-co-glycolic acid) of equal mass. A raw material for preparing the top coating is a combination of dibutyl hydroxy toluene and butyl hydroxy anisole of equal mass. A raw material for preparing the intermediate coating includes paclitaxel and a lipophilic material (DC-cholesterol, distearoyl phosphatidylcholine, and myristic acid).

A mass ratio of DC-cholesterol to distearoyl phosphatidylcholine to myristic acid is 1:3:4. A mass ratio of paclitaxel to the lipophilic material is 1:2. A mass ratio of the raw material for the bottom coating to paclitaxel is 2:4. A mass ratio of the raw material for the top coating to paclitaxel is 2:4. A loading amount of paclitaxel in the intermediate coating is 2 µg/mm².

The preparation method for the drug-coated balloon is as follows.
(1) Hydroxypropyl cellulose and poly(lactic-co-glycolic acid) are dissolved in ethanol to form a colorless transparent solution, i.e., a bottom coating solution.
(2) DC-cholesterol, distearoyl phosphatidylcholine, myristic acid, and paclitaxel are dissolved in n-heptane and sonicated to form a white suspension liquid, i.e., an intermediate coating solution.
(3) Dibutyl hydroxy toluene and butyl hydroxy anisole are dissolved in n-heptane to form a transparent solution, i.e., a top coating solution.
(4) The outer surface of the balloon body is sprayed with the bottom coating solution for two times, the intermediate coating solution for four times, and the top coating solution for four times in sequence by using an ultrasonic atomization spraying device, wherein the ultrasonic power is 2.0 W, the flow rate of the drug solution delivered by a syringe pump is 0.05 mL/min, the spraying temperature is 25°C, the spraying pressure is 0.2 MPa, and the spraying range is 20 mm.

### Example 4

This example provides a drug-coated balloon and a preparation method therefor. The drug-coated balloon includes a balloon body and a drug coating disposed on an outer surface of the balloon body. The drug coating includes a bottom coating, an intermediate coating, and a top coating which are stacked in sequence. The thicknesses of the bottom coating, the intermediate coating, and the top coating are respectively 2 µm, 6 µm, and 2 µm .

A raw material for preparing the bottom coating is a combination of polyethylene glycol and povidone of equal mass. A raw material for preparing the top coating is butyl hydroxy anisole. A raw material for preparing the intermediate coating includes paclitaxel and a lipophilic material (DC-cholesterol, distearoyl phosphatidylethanolamine).

A mass ratio of DC-cholesterol to distearoyl phosphatidylethanolamine is 1:1. A mass ratio of paclitaxel to the lipophilic material is 1:1. A mass ratio of the raw material for the bottom coating to paclitaxel is 2:1. A mass ratio of the raw material for the top coating to paclitaxel is 2:1. A loading amount of paclitaxel in the intermediate coating is 4 µg/mm².

The preparation method is as follows.
(1) Polyethylene glycol and povidone are dissolved in ethanol to form a colorless transparent solution, i.e., a bottom coating solution.
(2) DC-cholesterol, distearoyl phosphatidylethanolamine, and paclitaxel are dissolved in n-heptane and sonicated to form a white suspension liquid, i.e., an intermediate coating solution.
(3) Butyl hydroxy anisole is dissolved in n-heptane to form a transparent solution, i.e., a top coating solution.
(4) The outer surface of the balloon body is sprayed with the bottom coating solution for three times, the intermediate coating solution for five times, and the top coating solution for three times in sequence by using an ultrasonic atomization spraying device, wherein the ultrasonic power is 4.0 W, the flow rate of the drug solution delivered by a syringe pump is 0.2 mL/min, the spraying temperature is 30°C, the spraying pressure is 0.1 MPa, and the spraying range is 40 mm.

### Example 5

This example provides a drug-coated balloon and a preparation method therefor. The drug-coated balloon includes a balloon body and a drug coating disposed on an outer surface of the balloon body. The drug coating includes a bottom coating, an intermediate coating, and a top coating which are stacked in sequence. The thicknesses of the bottom coating, the intermediate coating, and the top coating are respectively 2 µm, 6 µm, and 2 µm .

A raw material for preparing the bottom coating is a combination of poly(lactic-co-glycolic acid) and polyethylene glycol of equal mass. A raw material for preparing the top coating is dibutyl hydroxy toluene. A raw material for preparing the intermediate coating includes rapamycin as a drug and DC-cholesterol and dipalmityl phosphatidylcholine as a lipophilic material.

A mass ratio of DC-cholesterol to dipalmityl phosphatidylcholine is 1:1. A mass ratio of the drug to the lipophilic material is 1:1. A mass ratio of the raw material for the bottom coating to the drug is 1:3. A mass ratio of the raw material for the top coating to the drug is 1:3. A loading amount of the drug in the intermediate coating is 3 µg/mm².

The preparation method is as follows.
(1) The raw material for preparing the bottom coating is dissolved in ethanol to form a colorless transparent solution, i.e., a bottom coating solution.
(2) The drug and the lipophilic material are dissolved in n-heptane and sonicated to form a white suspension liquid, i.e., an intermediate coating solution.
(3) The raw material for preparing the top coating is dissolved in n-heptane to form a transparent solution, i.e., a top coating solution.
(4) The outer surface of the balloon body is sprayed with the bottom coating solution for two times, the intermediate coating solution for six times, and the top coating solution for two times in sequence by using an ultrasonic atomization spraying device, wherein an ultrasonic power is 5.0 W, the flow rate of the drug solution delivered by a syringe pump is 0.15 mL/min, the spraying temperature is 25°C, the spraying pressure is 0.05 MPa, and the spraying range is 30 mm.

The applicant declares that although the drug-coated balloon and the preparation method therefor provided by the present application are illustrated in the present application by way of above-described embodiments, the present application is not limited to the above-described embodiments, i.e., it does not mean that the present application must rely on the above-described embodiments to be implemented. It should be clear to a person skilled in the art that any modification on the present application, equivalent substitution of each raw material of the product of the present application and the addition of auxiliary ingredients, and the choice of specific methods, etc., fall within the scope of protection and disclosure of the present application.

The preferred embodiments of the present application have been described in detail in the above. However, the present application is not limited to the specific details in the above embodiments. Within the scope of the technical concept of the present application, various simple modifications can be made to the technical solutions of the present application. These simple modifications all belong to the protection scope of the present application.

In addition, it should be noted that the various specific technical features described in the above specific embodiments can be combined in any suitable manner if there is no contradiction. In order to avoid unnecessary repetition, various possible combination manners will not be further described.

## Claims

1. A drug-coated balloon, comprising a balloon body and a drug coating disposed on an outer surface of the balloon body, wherein the drug coating comprises a bottom coating, an intermediate coating, and a top coating stacked in sequence.

2. The drug-coated balloon of claim 1, wherein the bottom coating comprises a polymer material, the intermediate coating comprises a drug and a lipophilic material, and the top coating comprises another polymer material.

3. The drug-coated balloon of claim 1, wherein the bottom coating comprises any one or a combination of at least two of poly(lactic-co-glycolic acid), polyethylene glycol, povidone, polyvinyl alcohol, methyl cellulose, hydroxymethyl cellulose, hydroxypropyl cellulose, or polycaprolactone; and/or
the top coating comprises any one or a combination of at least two of dibutyl hydroxy toluene, butyl hydroxy anisole, chitosan, or polycaprolactone.

4. The drug-coated balloon of claim 2, wherein the lipophilic material comprises any one or a combination of at least two of cholesterol, a phospholipid, or a fatty acid.

5. The drug-coated balloon of claim 4, wherein the lipophilic material comprises the cholesterol and the phospholipid, and a mass ratio of the cholesterol to the phospholipid is in a range from 0.2 to 10; or
the lipophilic material comprises the phospholipid and the fatty acid, and a mass ratio of the phospholipid to the fatty acid is in a range from 0.2 to 10; or
the lipophilic material comprises the cholesterol and the fatty acid, and a mass ratio of the cholesterol to the fatty acid is in a range from 0.04 to 100.

6. The drug-coated balloon of claim 2, wherein the drug comprises any one or a combination of at least two of sirolimus, a sirolimus derivative, paclitaxel, or a paclitaxel derivative.

7. The drug-coated balloon of claim 2, wherein a mass ratio of the drug to the lipophilic material is in a range from 0.1 to 10, a mass ratio of the polymer material of the bottom coating to the drug is in a range from 0.05 to 20, and a mass ratio of the polymer material of the top coating to the drug is in a range from 0.05 to 20.

8. The drug-coated balloon of claim 4, wherein the cholesterol comprises DC-cholesterol and/or cholesterol; and/or
the phospholipid comprises any one or a combination of at least two of 1,2-dioleoyl lecithin, dipalmitoyl phosphatidylcholine, distearoyl phosphatidylcholine, dimyristoyl phosphatidylcholine, 1-palmitoyl-2-oleoyl lecithin, distearoyl phosphatidylethanolamine, dioleoyl phosphatidylethanolamine, or dipalmitoyl phosphatidylethanolamine; and/or
the fatty acid comprises any one or a combination of at least two of palmitic acid, stearic acid, lauric acid, myristic acid, or arachidic acid.

9. A method for preparing the drug-coated balloon of any one of claims 1 to 8, comprising steps of:
(1) preparing a bottom coating solution, an intermediate coating solution, and a top coating solution respectively;
(2) spraying the bottom coating solution, the intermediate coating solution, and the top coating solution in sequence onto the surface of the balloon body to form the drug coating.

10. The method of claim 9, wherein in step (2), the spraying is performed by using an ultrasonic atomization spraying device and adopting a multi-layer stack spraying technology.
